# EUROPÄISCHE PATENTSCHRIFT

(11) **EP 0 835 250 B1**
(45) Veröffentlichungstag und Bekanntmachung des Hinweises auf die Patenterteilung: **22.05.2002**
(21) Anmeldenummer: 96920833.9
(22) Anmeldetag: 20.06.1996
(51) Int. Cl.: C07D 249/08, C07D 249/12, C07D 249/10, A01N 43/653

(54) **IMINOOXYBENZYLVERBINDUNGEN UND IHRE VERWENDUNG ZUR BEKÄMPFUNG VON TIERISCHEN SCHÄDLINGEN UND SCHADPILZEN**
IMINOOXYBENZYL COMPOUNDS AND THEIR USE AS PESTICIDES AND FUNGICIDES
COMPOSES D'IMINOOXYBENZYLE ET LEUR UTILISATION DANS LA LUTTE CONTRE LES ANIMAUX ET CHAMPIGNONS NUISIBLES

(30) Priorität: 27.06.1995 DE 19523288
(43) Veröffentlichungstag der Anmeldung: 15.04.1998
(73) Patentinhaber: BASF AKTIENGESELLSCHAFT, 67056 Ludwigshafen (DE)
(72) Erfinder: MÜLLER, Bernd, D-67227 Frankenthal (DE); SAUTER, Hubert, D-68167 Mannheim (DE); RÖHL, Franz, D-67105 Schifferstadt (DE); LORENZ, Gisela, D-67434 Hambach (DE); AMMERMANN, Eberhard, D-64646 Heppenheim (DE); STRATHMANN, Siegfried, D-67117 Limburgerhof (DE)
(86) Internationale Anmeldenummer: EP9602665
(87) Internationale Veröffentlichungsnummer: WO9701545

(56) Entgegenhaltungen:
- EP-A- 0 370 629
- EP-A- 0 414 153
- EP-A- 0 460 575
- EP-A- 0 463 488
- EP-A- 0 472 300
- WO-A-90/07493
- WO-A-92/13830

## Beschreibung

Die vorliegende Erfindung betrifft Iminooxybenzylverbindungen der Formel I 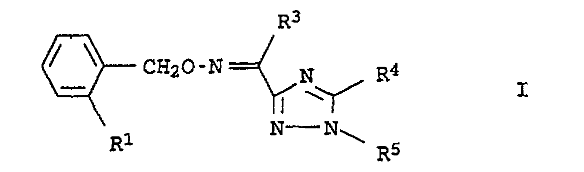 in der die Substituenten die folgende Bedeutung haben:
- R¹: C(CO₂R^{a})=CHR^{b};
- R^{a}, R^{b}: C₁-C₄-Alkyl;
- R³: Cyano, Halogen, C₁-C₄-Alkyl, Cyclopropyl, Trifluormethyl, Methoxy, Ethoxy oder Methylthio;
- R⁴: Wasserstoff, Cyano, Halogen, C₁-C₄-Alkyl, C₁-C₄-Halogenalkyl, C₁-C₄-Alkoxy, C₁-C₄-Halogenalkoxy oder C₁-C₄-Alkylthio;
- R⁵: Phenyl, welches ein bis drei der folgenden Reste tragen kann: Cyano, Halogen, C₁-C₄-Alkyl oder CRⁱⁱⁱ=NOR^{iv}; oder welches durch C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₁-C₆-Alkoxy, C₂-C₆-Alkenyloxy, C₂-C₆-Alkinyloxy, C₁-C₆-Halogenalkyloxy, C₂-C₆-Halogenalkenyloxy, C₂-C₆-Halogenalkinyloxy substituiert sein kann;
- Rⁱⁱⁱ: Wasserstoff, C₁-C₆-Alkyl, C₂-C₆-Alkenyl oder C₂-C₆-Alkinyl;
- R^{iv}: C₁-C₆-Alkyl, C₂-C₆-Alkenyl, C₂-C₆-Alkinyl oder Phenyl-C₁-C₆-alkyl.

Außerdem betrifft die Erfindung Verfahren zur Herstellung dieser Verbindungen sowie sie enthaltende Mittel und deren Verwendung zur Bekämpfung von tierischen Schädlingen und Schadpilzen.

In der Literatur werden Hetaryliminooxybenzylverbindungen mit fungizider Wirksamkeit in allgemeiner Form beschrieben (EP-A 370 629; EP-A 414 153; EP-A 460 575; EP-A 463 488; EP-A 472 300; EP-A 569 384; WO-A 90/07,493).

Der vorliegenden Erfindung lagen demgegenüber Verbindungen mit verbesserter Wirksamkeit als Aufgabe zugrunde.

Demgemäß wurden die eingangs definierten Verbindungen I gefunden. Außerdem wurden Verfahren zur Herstellung dieser Verbindungen sowie sie enthaltende Mittel und deren Verwendung zur Bekämpfung von tierischen Schädlingen und Schadpilzen gefunden.

Die Verbindungen I sind auf verschiedenen Wegen herstellbar. Besonders vorteilhaft erhält man die Verbindungen I dadurch, daß man eine Benzylverbindung der Formel II mit einem Oxim der Formel III oder dessen Salz umsetzt.

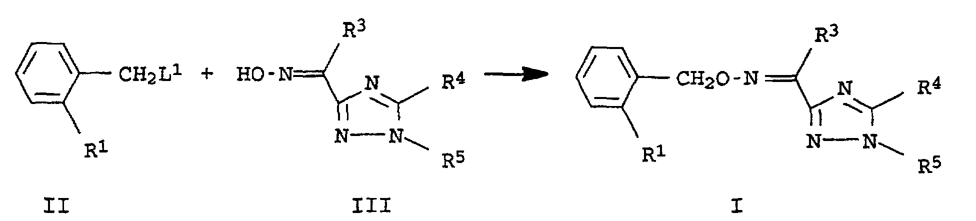

L¹ in der Formel II steht für eine Abgangsgruppe, d.h. eine nucleophil austauschbare Gruppe wie Halogen (z.B. Chlor, Brom und Iod), oder ein Alkyl- oder Arylsulfonat (z.B. Methylsulfonat, Trifluormethylsulfonat, Phenylsulfonat und 4-Methylsulfonat).

Die Oxime III können auch in Form ihrer Salze, z.B. mit anorganischen Säuren wie Hydrochloride, Hydrobromide, Hydrosulfate und Hydrophosponate, verwendet werden.

Die Umsetzung der Verbindungen II und III erfolgt üblicherweise bei Temperaturen von 0°C bis 80°C, vorzugsweise 20°C bis 60°C, in einem inerten Lösungsmittel in Gegenwart einer Base.

Geeignete Lösungsmittel sind aromatische Kohlenwasserstoffe wie Toluol, o-, m- und p-Xylol, halogenierte Kohlenwasserstoffe wie Methylenchlorid, Chloroform und Chlorbenzol, Ether wie Dieethylether, Diisopropylether, tert.-Butylmethylether, Dioxan, Anisol und Tetrahydrofuran, Nitrile wie Acetonitril und Propionitril, Alkohole wie Methanol, Ethanol, n-Propanol, i-Propanol, n-Butanol und tert.-Butanol, Ketone wie Aceton und Methylethylketon sowie Dimethylsulfoxid, Dimethylformamid, Dimethylacetamid, 1,3-Dimethylimidazolidin-2-on und 1,2-Dimethyltetrahydro-2(1H)-pyrimidin, vorzugsweise Methylenchlorid, Aceton, Toluol, tert.-Butylmethylether und Dimethylform-amid. Es können auch Gemische der genannten Lösungsmittel verwendet werden.

Als Basen kommen allgemein anorganische Verbindungen wie Alkalimetall- und Erdalkalimetallhydroxide (z.B. Lithiumhydroxid, Natriumhydroxid, Kaliumhydroxid und Calciumhydroxid), Alkalimetall- und Erdalkalimetalloxide (z.B. Lithiumoxid, Natriumoxid, Calciumoxid und Magnesiumoxid), Alkalimetall- und Erdalkalimetallhydride (z.B. Lithiumhydrid, Matriumhydrid, Kaliumhydrid und Calciumhydrid), Alkalimetallamide (z.B. Lithiumamid, Natriumamid und Kaliumamid), Alkalimetall- und Erdalkalimetallcarbonate (z.B. Lithiumcarbonat und Caiciumcarbonat) sowie Alkalimetallhydrogencarbonate (z.B. Natriumhydrogencarbonat), metallorganische Verbindungen, insbesondere Alkalimetallalkyle (z.B. Methyllithium, Butyllithium und Phenyllithium), Alkylmagnesiumhalogenide (z.B. Methylmagnesiumchlorid) sowie Alkalimetall- und Erdalkalimetallalkoholate (z.B. Natriummethanolat, Natriumethanolat, Kaliumethanolat, Kalium-tert.-Butanolat und Dimethoxymagnesium), außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht.

Besonders bevorzugt werden Natriumhydroxid, Kaliumcarbonat und Kalium-tert.-butanolat.

Die Basen werden im allgemeinen äquimolar, im Überschuß oder gegebenenfalls als Lösungsmittel verwendet.

Es kann für die Umsetzung vorteilhaft sein, eine katalytische Menge eines Kronenethers (z.B. 18-Krone-6 oder 15-Krone-5) zuzusetzen.

Die Umsetzung kann auch in Zweiphasensystemen bestehend aus einer Lösung von Alkali- oder Erdalkalihydroxiden oder -carbonaten in Wasser und einer organischen Phase (z.B. aromatische und/oder halogenierte Kohlenwasserstoffe) durchgeführt werden. Als Phasentransferkatalysatoren kommen hierbei beispielsweise Ammoniumhalogenide und -tetrafluoroborate (z.B. Benzyltriethylammoniumchlorid, Benzyltributylammoniumbromid, Tetrabutylammoniumchlorid, Hexadecyltrimethylammoniumbromid oder Tetrabutylammoniumtetrafluoroborat) sowie Phosphoniumhalogenide (z.B. Tetrabutylphosphoniumchlorid und Tetraphenylphosphoniumbromid) in Betracht.

Die für die Herstellung der Verbindungen I benötigten Ausgangsstoffe II sind aus der eingangs zitierten Literatur bekannt oder können nach den dort beschriebenen Methoden hergestellt werden.

Die Oxime III und ihre Herstellung sind in der zeitgleichen Anmeldung DE Pat. Anm. P 19 523 289.5 beschrieben.

Es kann für die Umsetzung vorteilhaft sein, zunächst das Oxim III mit der Base in das entsprechende Salz zu überführen, welche dann mit dem Benzylderivat II umgesetzt wird.

Die Verbindungen I können saure oder basische Zentren enthalten und dementsprechend Säureadditionsprodukte oder Basenadditionsprodukte oder Salze bilden.

Säuren für Säureadditionsprodukte sind u.a. Mineralsäuren (z.B. Halogenwasserstoffsäuren wie Chlorwasserstoff- und Bromwasserstoffsäure, Phosphorsäure, Schwefelsäure, Salpetersäure), organische Säuren (z.B. Ameisensäure, Essigsäure, Oxalsäure, Malonsäure, Milchsäure, Äpfelsäure, Bernsteinsäure, Weinsäure, Zitronensäure, Salicylsäure, p-Toluolsulfonsäure, Dodecylbenzolsulfonsäure) oder andere protonenacide Verbindugnen (z.B. Saccharin).

In entsprechender Weise erhält man die Verbindungen I auch dadurch, daß man einen Hydroxylaminether der Formel IV in einem inerten organischen Lösungsmittel mit einem Triazolylketon der Formel V umsetzt.

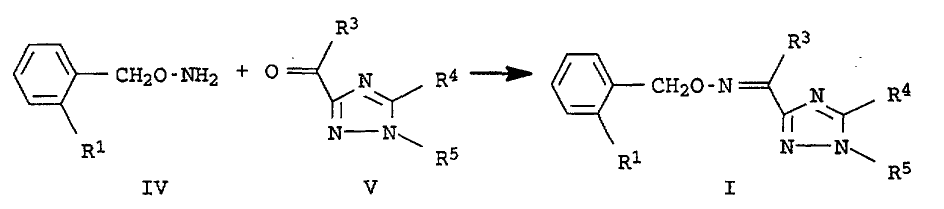

Diese Umsetzung verläuft im allgemeinen und im besonderen unter den für die Umsetzung von II mit III beschriebenen Bedingungen.

Außerdem kann die Umsetzung des Benzyloxyamins IV mit der Carbonylverbindung V auch unter neutralen oder sauren Bedingungen durchgeführt werden.

Als saure Katalysatoren kommen Mineralsäuren (z.B. Salzsäure, Schwefelsäure, Phosphorsäure und Salpetersäure) oder auch organische Säuren (z.B. Ameisensäure, Essigsäure, Propionsäure, Triethylamin-hydrochlorid, p-Toluolsulfonsäure, Methansulfonsäure, Citronensäure und saure Ionenaustauscher) in Betracht.

Nach einem weiteren Verfahren erhält man die Verbindungen I beispielsweise dadurch, daß man eine Benzylverbindung IIa analog dem vorstehend beschriebenen Verfahren mit einem Oxim der Formel III zum Benzylnitril VI umsetzt, VI zum Phenylessigsäureester VII hydrolisiert, VII zum α-Keto-phenylessigsaureester VIII oxidiert und VIII in an sich bekannter Weise zu I umsetzt.

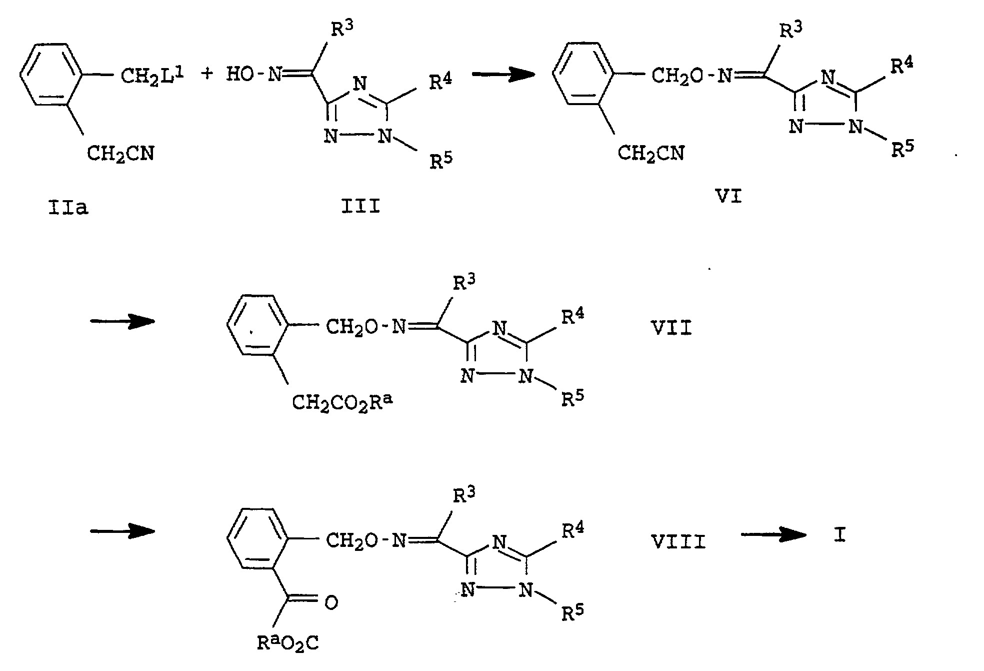

Die Hydrolyse von VI zu VII erfolgt im allgemeinen und im besonderen nach den in EP-A 493 711 beschriebenen Methoden.

Die Oxidation von VII zu VIII erfolgt ebenfalls im allgemeinen und im besonderen nach den in EP-A 493 711 oder nach den in Synth. Commun. 21, 2045 (1991) oder den in Synth. Commun. 11, 943 (1981) beschriebenen Methoden.

Die Umsetzung von VIII zu I erfolgt im allgemeinen und im besonderen nach den in den folgenden Schriften beschriebenen Methoden: R¹ ≡ C(CO₂R^{a})=CHOR^{b}: EP-A 203 608;

Säuren für Säureadditionsprodukte sind u.a. Mineralsäuren (z.B. Halogenwasserstoffsäuren wie Chlorwasserstoff- und Bromwasserstoffsäure, Phosphorsäure, Schwefelsäure, Salpetersäure), organische Säuren (z.B. Ameisensäure, Essigsäure, Oxalsäure, Malonsäure, Milchsäure, Äpfelsäure, Bernsteinsäure, Weinsäure, Zitronensäure, Salicylsäure, p-Toluolsulfonsäure, Dodecylbenzolsulfonsäure) oder andere protonenacide Verbindugnen (z.B. Saccharin).

Die Verbindungen I können in Bezug auf die C=N-Doppelbindung sowohl in der E- als auch in der Z-Konfiguration vorliegen. Beide Isomere können erfindungsgemäß gemeinsam oder getrennt angewendet werden. Im Hinblick auf die Wirkung gegen Schadpilze ist insbesondere dasjenige Isomer bevorzugt, in dem der Hydroxylamin-Sauerstoff und der Triazolylrest trans ständig sind.

Außerdem können die Verbindungen I aufgrund ihrer in R¹ enthaltenen Doppelbindung sowohl als E- als auch als Z-Isomere vorliegen. Beide Isomere können erfindungsgemäß gemeinsam oder getrennt angewendet werden. Im Hinblick auf die Wirkung gegen Schadpilze ist insbesondere dasjenige Isomer bevorzugt, in dem die Carbonylgruppe und der Rest R^{b} bzw. R^{b}O trans ständig sind.

Bei den in den vorstehenden Formeln angegebenen Definitionen der Symbole wurden z.T. Sammelbegriffe verwendet, die allgemein repräsentativ für die folgenden Substituenten stehen:
Halogen: Fluor, Chlor, Brom und Jod;
Alkyl: gesättigte, geradkettige oder verzweigte Kohlenwasserstoffreste mit vorzugsweise 1 bis 10 Kohlenstoffatomen, z.B. Methyl, Ethyl, Propyl, 1-Methylethyl, Butyl, 1-Methyl-propyl, 2-Methylpropyl und 1,1-Dimethylethyl;
Halogenalkyl: geradkettige oder verzweigte Alkylgruppen mit vorzugsweise 1 bis 10 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
Alkoxy: geradkettige oder verzweigte Alkylgruppen mit vorzugsweise 1 bis 10 Kohlenstoffatomen (wie vorstehend genannt), welche über ein Sauerstoffatom (-O-) an das Gerüst gebunden sind; Halogenalkoxy: geradkettige oder verzweigte Halogenalkylgruppen mit vorzugsweise 1 bis 10 Kohlenstoffatomen (wie vorstehend genannt), welche über ein Sauerstoffatom (-O-) an das Gerüst gebunden sind;
Alkylthio: geradkettige oder verzweigte Alkylgruppen mit vorzugsweise 1 bis 10 Kohlenstoffatomen (wie vorstehend genannt), welche über ein Schwefelatom (-S-) an das Gerüst gebunden sind; Halogenalkylthio: geradkettige oder verzweigte Halogenalkylgruppen mit vorzugsweise 1 bis 4 Kohlenstoffatomen (wie vorstehend genannt), welche über ein Schwefelatom (-S-) an das Gerüst gebunden sind;
Alkenyl: ungesättigte, geradkettige oder verzweigte Kohlenwasserstoffreste mit vorzugsweise 2 bis 10 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Di-methyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;
Alkenyloxy: geradkettige oder verzweigte Alkenylgruppen mit vorzugsweise 3 bis 10 Kohlenstoffatomen (wie vorstehend genannt), welche über ein Sauerstoffatom (-O-) an das Gerüst gebunden sind;
Alkinyl: geradkettige oder verzweigte Kohlenwasserstoffgruppen mit vorzugsweise 2 bis 10 Kohlenstoffatomen und einer Dreifachbindung in einer beliebigen Position, z.B. C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl- 1-pentinyl, 3-Methyl-4-pentinyl, 4-Methyl-1-pentinyl, 4-Methyl-2-pentinyl, 1,1-Dimethyl-2-butinyl, 1,1-Di-methyl-3-butinyl, 1,2-Dimethyl-3-butinyl, 2,2-Dimethyl-3-butinyl, 3,3-Dimethyl-l-butinyl, 1-Ethyl-2-butinyl, 1-Ethyl-3-butinyl, 2-Ethyl-3-butinyl und 1-Ethyl-1-methyl-2-propinyl;
Alkinyloxy: geradkettige oder verzweigte Alkinylgruppen mit vorzugsweise 3 bis 10 Kohlenstoffatomen (wie vorstehend genannt), welche über ein Sauerstoffatom (-O-) an das Gerüst gebunden sind;
Cycloalkyl: mono- oder bicyclische Kohlenwaserstoffreste mit vorzugsweise 3 bis 10 Kohlenstoffatomen, z.B. C₃-C₁₀-(Bi)cycloalkyl wie Cyclopropyl, Cyclobutyl, Cyclopentyl, Cyclohexyl, Cycloheptyl, Bornanyl, Norbornanyl, Dicyclohexyl, Bicyclo[3,3,0]octyl, Bicyclo[3,2,1]octyl, Bicyclo[2,2,2]octyl oder Bicyclo[3,3,1]nonyl;
unsubstituiertes oder durch übliche Gruppen substituiertes Phenyl enthalten und die Alkylgruppen in diesen Resten vorzugsweise 1 bis 6 Kohlenstoffatome, insbesondere 1 bis 4 Kohlenstoffatome enthalten,
und oder einen oder zwei (insbesondere einen) der folgenden Reste

Formyl oder CRⁱⁱⁱ=NOR^{iv}, wobei Rⁱⁱⁱ Wasserstoff, Alkyl, Alkenyl oder Alkinyl und R^{iv} Alkyl, Alkenyl, Alkinyl und Arylalkyl bedeutet und wobei die genannten Alkylgruppen vorzugsweise 1 bis 6 Kohlenstoffatome, insbesondere 1 bis 4 Kohlenstoffatome, und die Alkenyl- bzw. Alkinylgruppen vorzugsweise 2 bis 6 Kohlenstoffatome enthalten und Aryl insbesondere Phenyl bedeutet, welches unsubstituiert ist oder durch übliche Gruppen substituiert sein kann,
tragen können.

Unter *"üblichen Gruppen"*, die als Substituenten von R⁴ in Betracht kommen, sind die vorstehend als mögliche Substituenten von cyclischen Systemen genannten Reste zu verstehen.

Im Hinblick auf ihre biologische Wirksamkeit sind Verbindungen I bevorzugt, in denen R^{a} und R^{b} C₁-C₂-Alkyl, insbesondere Methyl, bedeuten.

Außerdem werden Verbindungen I bevorzugt, in denen R¹ C (CO₂R^{a}) =CHR^{b} bedeutet.

Erfindungsgemäß sind Verbindungen I, in denen R³ für C₁-C₄-Alkyl, insbesondere Methyl, Cyano, Trifluormethyl, Halogen, Methoxy, Ethoxy und Methylthio steht.

Erfindungsgemäß sind Verbindungen I, in denen R⁴ für Wasserstoff, Cyano, Halogen, C₁-C₄-Alkyl, C₁-C₄-Halogenalkyl, C₁-C₄-Alkoxy, C₁-C₄-Halogenalkoxy oder C₁-C₄-Alkylthio steht.

Erfindungsgemäß sind Verbindungen I, in denen R⁵ für ggf. subst. Phenyl steht.

Außerdem werden Verbindungen I bevorzugt, in denen R⁵ ein bis drei der folgenden Gruppen trägt: Cyano, Halogen, C₁-C₄-Alkyl und CRⁱⁱⁱ=NOR^{iv}.

Desweiteren werden Verbindugnen I bevorzugt, in denen R⁵ durch C₁-C₆-Alkyl, C₂-C₆-Alkenyl, C₂-C₆-Alkinyl, C₁-C₆-Halogenalkyl, C₂-C₆-Halogenalkenyl und C₂-C₆-Halogenalkinyl substituiert ist.

Gleichermaßen besonders bevorzugt sind Verbindungen I, in denen R⁵ durch C₁-C₆-Alkoxy, C₂-C₆-Alkenyloxy, C₂-C₆-Alkinyloxy, C₁-C₆-Halogenalkyloxy, C₂-C₆-Halogenalkenyloxy und C₂-C₆-Halogenalkinyloxy substituiert ist.

Insbesondere sind im Hinblick auf ihre Verwendung die in den folgenden Tabellen zusammengestellten Verbindungen I bevorzugt. Die in den Tabellen für einen Substituenten genannten Gruppen stellen außerdem für sich betrachtet (unabhängig von der Kombination, in der sie genannt sind) eine besonders bevorzugte Ausgestaltung des betreffenden Substituenten dar.

### Tabelle 1

Verbindungen der Formel I.A, in denen R³ für Methyl steht, R⁴ Wasserstoff bedeutet und Rₓ für eine Verbindung jeweils einer Gruppe der Tabelle A entspricht

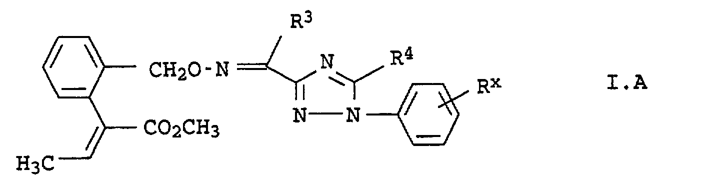

### Tabelle 5

Verbindungen der Formel I.A, in denen R³ für Methyl steht, R⁴ Methyl bedeutet und Rₓ für eine Verbindung jeweils einer Gruppe der Tabelle A entspricht

### Tabelle 9

Verbindungen der Formel I.A, in denen R³ für Methyl steht, R⁴ Trifluormethyl bedeutet und Rₓ für eine Verbindung jeweils einer Gruppe der Tabelle A entspricht

### Tabelle 13

Verbindungen der Formel I.A, in denen R³ für Methyl steht, R⁴ Methoxy bedeutet und Rₓ für eine Verbindung jeweils einer Gruppe der Tabelle A entspricht

### Tabelle 17

Verbindungen der Formel I.A, in denen R³ für Trifluormethyl steht, R⁴ Wasserstoff bedeutet und Rₓ für eine Verbindung jeweils einer Gruppe der Tabelle A entspricht

### Tabelle 21

Verbindungen der Formel I.A, in denen R³ für Trifluormethyl steht, R⁴ Methyl bedeutet und Rₓ für eine Verbindung jeweils einer Gruppe der Tabelle A entspricht

### Tabelle 25

Verbindungen der Formel I.A, in denen R³ für Trifluormethyl steht, R⁴ Trifluormethyl bedeutet und Rₓ für eine Verbindung jeweils einer Gruppe der Tabelle A entspricht

### Tabelle 29

Verbindungen der Formel I.A, in denen R³ für Trifluormethyl steht, R⁴ Methoxy bedeutet und Rₓ für eine Verbindung jeweils einer Gruppe der Tabelle A entspricht

### Tabelle 33

Verbindungen der Formel I.A, in denen R³ für Ethyl steht, R⁴ Wasserstoff bedeutet und Rₓ für eine Verbindung jeweils einer Gruppe der Tabelle A entspricht

### Tabelle 37

Verbindungen der Formel I.A, in denen R³ für Ethyl steht, R⁴ Methyl bedeutet und Rₓ für eine Verbindung jeweils einer Gruppe der Tabelle A entspricht

### Tabelle 41

Verbindungen der Formel I.A, in denen R³ für Ethyl steht, R⁴ Trifluormethyl bedeutet und Rₓ für eine Verbindung jeweils einer Gruppe der Tabelle A entspricht

### Tabelle 45

Verbindungen der Formel I.A, in denen R³ für Ethyl steht, R⁴ Methoxy bedeutet und Rₓ für eine Verbindung jeweils einer Gruppe der Tabelle A entspricht

### Tabelle 49

Verbindungen der Formel I.A, in denen R³ für Cyclopropyl steht, R⁴ Wasserstoff bedeutet und Rₓ für eine Verbindung jeweils einer Gruppe der Tabelle A entspricht

### Tabelle 53

Verbindungen der Formel I.A, in denen R³ für Cyclopropyl steht, R⁴ Methyl bedeutet und Rₓ für eine Verbindung jeweils einer Gruppe der Tabelle A entspricht

### Tabelle 57

Verbindungen der Formel I.A, in denen R³ für Cyclopropyl steht, R⁴ Trifluormethyl bedeutet und Rₓ für eine Verbindung jeweils einer Gruppe der Tabelle A entspricht

### Tabelle 61

Verbindungen der Formel I.A, in denen R³ für Cyclopropyl steht, R⁴ Methoxy bedeutet und Rₓ für eine Verbindung jeweils einer Gruppe der Tabelle A entspricht

### Tabelle 65

Verbindungen der Formel I.A, in denen R³ für Methyl steht, R⁴ Ethoxy bedeutet und Rₓ für eine Verbindung jeweils einer Gruppe der Tabelle A entspricht

### Tabelle 69

Verbindungen der Formel I.A, in denen R³ für Trifluormethyl steht, R⁴ Ethoxy bedeutet und Rₓ für eine Verbindung jeweils einer Gruppe der Tabelle A entspricht

### Tabelle 73

Verbindungen der Formel I.A, in denen R³ für Ethyl steht, R⁴ Ethoxy bedeutet und Rₓ für eine Verbindung jeweils einer Gruppe der Tabelle A entspricht

### Tabelle 77

Verbindungen der Formel I.A, in denen R³ für Cyclopropyl steht, R⁴ Ethoxy bedeutet und Rₓ für eine Verbindung jeweils einer Gruppe der Tabelle A entspricht

**Tabelle A**

| Nr. | R^{x} |
|---|---|
| 11 | H |
| 12 | 2-F |
| 13 | 3-F |
| 14 | 4-F |
| 15 | 2,4-F₂ |
| 16 | 2,4,6-F₃ |
| 17 | 2,3-F₂ |
| 18 | 2-Cl |
| 19 | 3-Cl |
| 20 | 4-Cl |
| 21 | 2,3-Cl₂ |
| 22 | 2,4-Cl₂ |
| 23 | 2,5-Cl₂ |
| 24 | 2,6-Cl₂ |
| 25 | 3,4-Cl₂ |
| 26 | 3,5-Cl₂ |
| 27 | 2,3,4-Cl₃ |
| 28 | 2,3,5-Cl₃ |
| 29 | 2,3,6-Cl₃ |
| 30 | 2,4,5-Cl₃ |
| 31 | 2,4,6-Cl₃ |
| 32 | 3,4,5-Cl₃ |
| 33 | 2-Br |
| 34 | 3-Br |
| Nr. | R^{x} |
| 35 | 4-Br |
| 36 | 2,4-Br₂ |
| 37 | 2,5-Br₂ |
| 38 | 2,6-Br₂ |
| 39 | 2,4,6-Br₃ |
| 40 | 2-J |
| 41 | 3-J |
| 42 | 4-J |
| 43 | 2,4-J₂ |
| 44 | 2-C1, 3-F |
| 45 | 2-Cl, 4-F |
| 46 | 2-Cl, 5-F |
| 47 | 2-Cl, 6-F |
| 48 | 2-Cl, 3-Br |
| 49 | 2-Cl, 4-Br |
| 50 | 2-Cl, 5-Br |
| 51 | 2-Cl, 6-Br |
| 52 | 2-Br, 3-Cl |
| 53 | 2-Br, 4-Cl |
| 54 | 2-Br, 5-Cl |
| 55 | 2-Br, 3-F |
| 56 | 2-Br, 4-F |
| 57 | 2-Br, 5-F |
| 58 | 2-Br, 6-F |
| 59 | 2-F, 3-Cl |
| 60 | 2-F, 4-Cl |
| 61 | 2-F, 5-Cl |
| 62 | 3-Cl, 4-F |
| 63 | 3-Cl, 5-F |
| 64 | 3-Cl, 4-Br |
| 65 | 3-Cl, 5-Br |
| 66 | 3-F, 4-Cl |
| 67 | 3-F, 4-Br |
| 68 | 3-Br, 4-Cl |
| 69 | 3-Br, 4-F |
| 70 | 2,6-Cl₂, 4-Br |
| 71 | 2-CH₃ |
| Nr. | R^{x} |
| 72 | 3-CH₃ |
| 73 | 4-CH₃ |
| 74 | 2,3-(CH₃)₂ |
| 75 | 2,4-(CH₃)₂ |
| 76 | 2,5-(CH₃)₂ |
| 77 | 2,6-(CH₃)₂ |
| 78 | 3,4-(CH₃)₂ |
| 79 | 3,5-(CH₃)₂ |
| 80 | 2,3,5-(CH₃)₃ |
| 81 | 2,3,4-(CH₃)₃ |
| 82 | 2,3,6-(CH₃)₃ |
| 83 | 2,4,5-(CH₃)₃ |
| 84 | 2,4,6-(CH₃)₃ |
| 85 | 3,4,5-(CH₃)₃ |
| 86 | 2-C₂H₅ |
| 87 | 3-C₂H₅ |
| 88 | 4-C₂H₅ |
| 89 | 2,4-(C₂H₅)₅ |
| 90 | 2,6-(C₂H₅)₂ |
| 91 | 3,5-(C₂H₅)₂ |
| 92 | 2,4,6-(C₂H₅)₃ |
| 93 | 2-n-C₃H₇ |
| 94 | 3-n-C₃H₇ |
| 95 | 4-n-C₃H₇ |
| 96 | 2-i-C₃H₇ |
| 97 | 3-i-C₃H₇ |
| 98 | 4-i-C₃H₇ |
| 99 | 2,4-(i-C₃H₇)₂ |
| 100 | 2,6-(i-C₃H₇)₂ |
| 101 | 3,5-(i-C₃H₇)₂ |
| 102 | 2-s-C₄H₉ |
| 103 | 3-s-C₄H₉ |
| 104 | 4-s-C₄H₉ |
| 105 | 2-t-C₄H₉ |
| 106 | 3-t-C₄H₉ |
| 107 | 4-t-C₄H₉ |
| 108 | 4-n-C₉H₁₉ |
| Nr. | R^{x} |
| 109 | 2-CH₃, 4-t-C₄H₉ |
| 110 | 2-CH₃, 6-t-C₄H₉ |
| 111 | 2-CH₃, 4-i-C₃H₇ |
| 112 | 2-CH₃, 5-i-C₃H₇ |
| 113 | 3-CH₃, 4-i-C₃H₇ |
| 114 | 2-OCH₃ |
| 115 | 3-OCH₃ |
| 116 | 4-OCH₃ |
| 117 | 2-OC₂H₅ |
| 118 | 3-O-C₂H₅ |
| 119 | 4-O-C₂H₅ |
| 120 | 2-O-n-C₃H₇ |
| 121 | 3-O-n-C₃H₇ |
| 122 | 4-O-n-C₃H₇ |
| 123 | 2-O-i-C₃H₇ |
| 124 | 3-O-i-C₃H₇ |
| 125 | 4-O-i-C₃H₇ |
| 126 | 2-O-n-C₆H₁₃ |
| 127 | 3-O-n-C₆H₁₃ |
| 128 | 4-O-n-C₆H₁₃ |
| 129 | 2-O-t-C₄H₉ |
| 130 | 3-O-t-C₄H₉ |
| 131 | 4-O-t-C₄H₉ |
| 132 | 2,4-(CH₃)₂, 6-F |
| 133 | 2,4-(CH₃)₂, 6-Cl |
| 134 | 2,4-(CH₃)₂, 6-Br |
| 135 | 2-i-C₃H₇, 4-Cl, 5-CH₃ |
| 136 | 2-CH₃, 5-i-C₃H₇, 4-Cl |
| 137 | 2-CH₃-4-(CH₃-C=NOCH₃) |
| 138 | 2-CH₃-4-(CH₃-C=NOC₂H₅) |
| 139 | 2-CH₃-4-(CH₃-C=NO-n-C₃H₇) |
| 140 | 2-CH₃-4-(CH₃-C=NO-i-C₃H₇) |
| 141 | 2,5-(CH₃)₂-4-(CH₃-C=NOCH₃) |
| 142 | 2,5-(CH₃)₂-4-(CH₃-C=NOC₂H₅) |
| 143 | 2,5-(CH₃-4-(CH₃-C=NO-n-C₃H₇) |
| 144 | 2,5-(CH₃)₂-4-(CH₃-C=NO-i-C₃H₇) |
| 145 | 2-CH₃, 5-Br |
| Nr. | R^{x} |
| 146 | 2-CH₃, 6-Br |
| 147 | 2-Cl, 3-CH₃ |
| 148 | 2-Cl, 4-CH₃ |
| 149 | 2-Cl, 5-CH₃ |
| 150 | 2-F, 3-CH₃ |
| 151 | 2-F, 4-CH₃ |
| 152 | 2-F, 5-CH₃ |
| 153 | 2-Br, 3-CH₃ |
| 154 | 2-Br, 4-CH₃ |
| 155 | 2-Br, 5-CH₃ |
| 156 | 3-CH₃, 4-Cl |
| 157 | 3-CH₃, 5-Cl |
| 158 | 3-CH₃, 4-F |
| 159 | 3-CH₃, 5-F |
| 160 | 3-CH₃, 4-Br |
| 161 | 3-CH₃, 5-Br |
| 162 | 3-F, 4-CH₃ |
| 163 | 3-Cl, 4-CH₃ |
| 164 | 3-Br, 4-CH₃ |
| 165 | 2-Cl, 4,5-(CH₃)₂ |
| 166 | 2-Br, 4,5-(CH₃)₂ |
| 167 | 2-Cl, 3,5-(CH₃)₂ |
| 168 | 2-Br, 3,5-(CH₃)₂ |
| 169 | 2,6-Cl₂, 4-CH₃ |
| 170 | 2,6-F₂, 4-CH₃ |
| 171 | 2,6-Br₂, 4-CH3 |
| 172 | 2,4-Br₂, 6-CH₃ |
| 173 | 2,4-F₂, 6-CH₃ |
| 174 | 2,4-Br₂, 6-CH₃ |
| 175 | 2,6-(CH₃)₂, 4-F |
| 176 | 2,6-(CH₃)₂, 4-Cl |
| 177 | 2,6-(CH₃)₂, 4-Br |
| 178 | 3,5-(CH₃)₂, 4-F |
| 179 | 3,5-(CH₃)₂, 4-Cl |
| 180 | 3,5-(CH₃)₂, 4-Br |
| 181 | 2-CF₃ |
| 182 | 3-CF₃ |
| Nr. | R^{x} |
| 183 | 4-CF₃ |
| 184 | 2-OCF₃ |
| 185 | 3-OCF₃ |
| 186 | 4-OCF₃ |
| 187 | 3-OCH₂CHF₂ |
| 188 | 2-CN |
| 189 | 3-CN |
| 190 | 4-CN |
| 191 | 2-CH₃, 3-Cl |
| 192 | 2-CH3, 4-Cl |
| 193 | 2-CH₃, 5-Cl |
| 194 | 2-CH₃, 6-Cl |
| 195 | 2-CH₃, 3-F |
| 196 | 2-CH₃, 4-F |
| 197 | 2-CH₃, 5-F |
| 198 | 2-CH₃, 6-F |
| 199 | 2-CH₃, 3-Br |
| 200 | 2-CH₃, 4-Br |
| 251 | 3,5-F₂ |

Die erfindungsgemäßen Verbindungen der Formel I eignen sich zur Bekämpfung von Schadpilzen und von tierischen Schädlingen aus der Klasse der Insekten, Spinnentiere und Nematoden. Sie können im Pflanzenschutz sowie auf dem Hygiene-, Vorratsschutz- und Veterinärsektor als Fungizide und Schädlingsbekämpfungsmittel eingesetzt werden.

Zu den schädlichen Insekten gehören:
aus der Ordnung der Schmetterlinge (Lepidoptera) beispielsweise Adoxophyes orana, Agrotis ypsilon, Agrotis segetum, Alabama argillacea, Anticarsia gemmatalis, Argyresthia conjugella, Autographa gamma, Cacoecia murinana, Capua reticulana, Choristoneura fumiferana, Chilo partellus, Choristoneura occidentalis, Cirphis unipuncta, Cnaphalocrocis medinalis, Crocidolomia binotalis, Cydia pomonella, Dendrolimus pini, Diaphania nitidalis, Diatraea grandiosella, Earias insulana, Elasmopalpus lignosellus, Eupoecilia ambiguella, Feltia subterranea, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera scitella, Lithocolletis blancardella, Lobesia botrana, Loxostege sticticalis, Lymantria dispar, Lymantria monacha, Lyonetia clerkella, Manduca sexta, Malacosoma neustria, Mamestra brassicae, Mocis repanda, Operophthera brumata, Orgyia pseudotsugata, Ostrinia nubilalis, Pandemis heparana, Panolis flammea, Pectinophora gossypiella, Phthorimaea operculella, Phyllocnistis citrella, Pieris brassicae, Plathypena scabra, Platynota stultana, Plutella xylostella, Prays citri, Prays oleae, Prodenia sunia, Prodenia ornithogalli, Pseudoplusia includens, Rhyacionia frustrana, Scrobipalpula absoluta, Sesamia inferens, Sparganothis pilleriana, Spodoptera frugiperda, Spodoptera littoralis, Spodoptera litura, Syllepta derogata, Synanthedon myopaeformis, Thaumatopoea pityocampa, Tortrix viridana, Trichoplusia ni, Tryporyza incertulas, Zeiraphera canadensis, ferner Galleria mellonella und Sitotroga cerealella, Ephestia cautella, Tineola bisselliella;
aus der Ordnung der Käfer (Coleoptera) beispielsweise Agriotes lineatus, Agriotes obscurus, Anthonomus grandis, Anthonomus pomorum, Apion vorax, Atomaria linearis, Blastophagus piniperda, Cassida nebulosa, Cerotoma trifurcata, Ceuthorhynchus assimilis, Ceuthorhynchus napi, Chaetocnema tibialis, Conoderus vespertinus, Crioceris asparagi, Dendroctonus refipennis, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllopertha horticola, Phyllophaga sp., Phyllotreta chrysocephala, Phyllotreta nemorum, Phyllotreta striolata, Popillia japonica, Psylliodes napi, Scolytus intricatus, Sitona lineatus, ferner Bruchus rufimanus, Bruchus pisorum, Bruchus lentis, Sitophilus granaria, Lasioderma serricorne, Oryzaephilus surinamensis, Rhyzopertha dominica, Sitophilus oryzae, Tribolium castaneum, Trogoderma granarium, Zabrotes subfasciatus;
aus der Ordnung der Zweiflügler (Diptera) beispielsweise Anastrepha ludens, Ceratitis capitata, Contarinia sorghicola, Dacus cucurbitae, Dacus oleae, Dasineura brassicae, Delia coarctata, Delia radicum, Hydrellia griseola, Hylemyia platura, Liriomyza sativae, Liriomyza trifolii, Mayetiola destructor, Orseolia oryzae, Oscinella frit, Pegomya hyoscyami, Phorbia antiqua, Phorbia brassicae, Phorbia coarctata, Rhagoletis cerasi, Rhagoletis pomonella, Tipula oleracea, Tipula paludosa, ferner Aedes aegypti, Aedes vexans, Anopheles maculipennis, Chrysomya bezziana, Chrysomya hominivorax, Chrysomya macellaria, Cordylobia anthropophaga, Culex pipiens, Fannia canicularis, Gasterophilus intestinalis, Glossina morsitans, Haematobia irritans, Haplodiplosis equestris, Hypoderma lineata, Lucilia caprina, Lucilia cuprina, Lucilia sericata, Musca domestica, Muscina stabulans, Oestrus ovis, Tabanus bovinus, Simulium damnosum;
aus der Ordnung der Thripse (Thysanoptera) beispielsweise Frankliniella fusca, Frankliniella occidentalis, Frankliniella tritici, Haplothrips tritici, Scirtothrips citri, Thrips oryzae, Thrips palmi, Thrips tabaci;
aus der Ordnung der Hautflügler (Hymenoptera) beispielsweise Athalia rosae, Atta cephalotes, Atta sexdens, Atta texana, Hoplocampa minuta, Hoplocampa testudinea, Iridomyrmes humilis, Iridomyrmex purpureus, Monomorium pharaonis, Solenopsis geminata, Solenopsis invicta, Solenopsis richteri;
aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus hesperus, Lygus lineolaris, Lygus pratensis, Nezara viridula, Piesma quadrata, Solubea insularis, Thyanta perditor;
aus der Ordnung der Pflanzensauger (Homoptera) beispielsweise Acyrthosiphon onobrychis, Acyrthosiphon pisum, Adelges laricis, Aonidiella aurantii, Aphidula nasturtii, Aphis fabae, Aphis gossypii, Aphis pomi, Aulacorthum solani, Bemisia tabaci, Brachycaudus cardui, Brevicoryne brassicae, Dalbulus maidis, Dreyfusia nordmannianae, Dreyfusia piceae, Dysaphis radicola, Empoasca fabae, Eriosoma lanigerum, Laodelphax striatella, Macrosiphum avenae, Macrosiphum euphorbiae, Macrosiphon rosae, Megoura viciae, Metopolophium dirhodum, Myzus persicae, Myzus cerasi, Nephotettix cincticeps, Nilaparvata lugens, Perkinsiella saccharicida, Phorodon humuli, Planococcus citri, Psylla mali, Psylla piri, Psylla pyricol, Quadraspidiotus perniciosus, Rhopalosiphum maidis, Saissetia oleae, Schizaphis graminum, Selenaspidus articulatus, Sitobion avenae, Sogatella furcifera, Toxoptera citricida, Trialeurodes abutilonea, Trialeurodes vaporariorum, Viteus vitifolii;
aus der Ordnung der Termiten (Isoptera) beispielsweise Calotermes flavicollis, Leucotermes flavipes, Macrotermes subhyalinus, Odontotermes formosanus, Reticulitermes lucifugus, Termes natalensis;
aus der Ordnung der Geradflügler (Orthoptera) beispielsweise Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Schistocerca americana, Schistocerca peregrina, Stauronotus maroccanus, Schistocerca gregaria, ferner Acheta domestica, Blatta orientalis, Blattella germanica, Periplaneta americana;
aus der Ordnung der Arachnoidea beispielsweise phytophage Milben wie Aculops lycopersicae, Aculops pelekassi, Aculus schlechtendali, Brevipalpus phoenicis, Bryobia praetiosa, Eotetranychus carpini, Eutetranychus banksii, Eriophyes sheldoni, Oligonychus pratensis, Panonychus ulmi, Panonychus citri, Phyllocoptruta oleivora, Polyphagotarsonemus latus, Tarsonemus pallidus, Tetranychus cinnabarinus, Tetranychus kanzawai, Tetranchus pacificus, Tetranychus urticae, Zecken wie Amblyomma americanum, Amblyomma variegatum, Argas persicus, Boophilus annulatus, Boophilus decoloratus, Boophilus microplus, Dermacentor silvarum, Hyalomma truncatum, Ixodes ricinus, Ixodes rubicundus, Ornithodorus moubata, Otobius megnini, Rhipicephalus appendiculatus und Rhipicephalus evertsi sowie tierparasitische Milben wie Dermanyssus gallinae, Psoroptes ovis und Sarcoptes scabiei;
aus der Klasse der Nematoden beispielsweise Wurzelgallennematoden, z.B. Meloidogyne hapla, Meloidogyne incognita, Meloidogyne javanica, zystenbildende Nematoden, z.B. Globodera pallida, Globodera rostochiensis, Heterodera avenae, Heterodera glycines, Heterodera schachtii, migratorische Endoparasiten und semi-endoparasitische Nematoden, z.B. Heliocotylenchus multicinctus, Hirschmanniella oryzae, Hoplolaimus spp, Pratylenchus brachyurus, Pratylenchus fallax, Pratylenchus penetrans, Pratylenchus vulnus, Radopholus similis, Rotylenchus reniformis, Scutellonema bradys, Tylenchulus semipenetrans, Stock- und Blattnematoden z.B. Anguina tritici, Aphelenchoides besseyi, Ditylenchus angustus, Ditylenchus dipsaci, Virusvektoren, z.B. Longidorus spp, Trichodorus christei, Trichodorus viruliferus, Xiphinema index, Xiphinema mediterraneum.

Die Verbindungen I können als solche, in Form ihrer Formulierungen oder den daraus bereiteten Anwendungsformen, z.B. in Form von direkt versprühbaren Lösungen, Pulvern, Suspensionen oder Dispersionen, Emulsionen, öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Als Fungizide sind die Verbindungen der Formel I z.T. systemisch wirksam. Sie können als Blatt- und Bodenfungizide gegen ein breites Spektrum von pflanzenpathogenen Pilzen, insbesondere aus der Klasse der Ascomyceten, Deuteromyceten, Phycomyceten und Basidiomyceten eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Rasen, Baumwolle, Soja, Kaffee, Zukkerrohr, Wein, Obst- und Zierpflanzen und Gemüsepflanzen wie Gurken, Bohnen und Kürbisgewächsen, sowie an den Samen dieser Pflanzen.

Speziell eignen sich die Verbindungen I zur Bekämpfung folgender Pflanzenkrankheiten:
* Erysiphe graminis (echter Mehltau) in Getreide,
* Erysiphe cichoracearum und Sphaerotheca fuliginea an Kürbisgewächsen,
* Podosphaera leucotricha an Äpfeln,
* Uncinula necator an Reben,
* Puccinia-Arten an Getreide,
* Rhizoctonia-Arten an Baumwolle und Rasen,
* Ustilago-Arten an Getreide und Zuckerrohr,
* Venturia inaequalis (Schorf) an Äpfeln,
* Helminthosporium-Arten an Getreide,
* Septoria nodorum an Weizen,
* Botrytis cinerea (Grauschimmel) an Erdbeeren, Reben,
* Cercospora arachidicola an Erdnüssen,
* Pseudocercosporella herpotrichoides an Weizen, Gerste,
* Pyricularia oryzae an Reis,
* Phytophthora infestans an Kartoffeln und Tomaten,
* Fusarium- und Verticillium-Arten an verschiedenen Pflanzen,
* Plasmopara viticola an Reben,
* Alternaria-Arten an Gemüse und Obst.

Die neuen Verbindungen können auch im Materialschutz z.B. zum Schutz von Holz, Papier und Textilien eingesetzt werden, z.B. gegen Paecilomyces variotii.

Sie können in die üblichen Formulierungen übergeführt werden, wie Lösungen, Emulsionen, Suspensionen, Stäube, Pulver, Pasten oder Granulate. Die Anwendungformen richten sich dabei nach dem jeweiligen Verwendungszweck; sie sollten in jedem Fall möglichst die feinste Verteilung der Wirkstoffe gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können.

Als Hilfsstoffe kommen dafür im wesentlichen in Betracht:
- Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser;
- Trägerstoffe wie natürliche Gesteinsmehle (z.B. Kaoline, Tonerden, Talkum, Kreide) und synthetische Gesteinsmehle (z.B. hochdisperse Kieselsäure, Silikate);
- Emulgiermittel wie nichtionogene und anionische Emulgatoren (z.B. Polyoxyethylen-Fettalkohol-Ether, Alkylsulfonate und Arylsulfonate) und
- Dispergiermittel wie Ligninsulfit-Ablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Lauryletherund Fettalkoholsulfaten, sowie Salze sulfatierter Hexa-, Heptaund Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Ö1 oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden.

Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe. Die Wirkstoffkonzentrationen in den anwendungsfertigen Zubereitungen können in größeren Bereichen variiert werden.

Ganz allgemein enthalten die Mittel zwischen 0,0001 und 95 Gew.-% Wirkstoff.

Formulierungen mit mehr als 95 Gew.-% Wirkstoff können mit gutem Erfolg im Ultra-Low-VolumeVerfahren (ULV) ausgebracht werden, wobei sogar der Wirksoff ohne Zusätze verwendet werden kann.

Für die Anwendung als Fungizide empfehlen sich Konzentrationen zwischen 0,01 und 95 Gew.%, vorzugsweise zwischen 0,5 und 90 Gew.%, Wirkstoff. Für die Anwendung als Insektizide kommen Formulierungen mit 0,0001 bis 10 Gew.%, vorzugsweise 0,01 bis 1 Gew.% Wirkstoff, in Betracht.

Die Wirkstoffe werden normalerweise in einer Reinheit von 90 % bis 100 %, vorzugsweise 95 % bis 100 % (nach NMR-Spektrum) eingesetzt.

Beispiele für solche Zubereitungen sind:
I. eine Lösung aus 90 Gew.-Teilen einer erfindungsgemäßen Verbindung I und 10 Gew.-Teilen N-Methyl-α-pyrrolidon, die zur Anwendung in Form kleinster Tropfen geeignet ist;
II. eine Lösung von 20 Gew.-Teilen einer erfindungsgemäßen Verbindung I in einer Mischung aus 80 Gew.-Teilen alkyliertem Benzol, 10 Gew.-Teilen des Anlagerungsproduktes von 8 bis 10 Mol Ethylenoxid an 1 Mol Ölsäure-N-monoethanolamid, 5 Gew.Teilen Caiciumsalz der Dodecylbenzolsulfonsäure, 5 Gew.-Teilen des Anlagerungsproduktes von 40 Mol Ethylen oxid an 1 Mol Ricinusöl; durch feines Verteilen der Formulierung in Wasser erhält man eine Dispersion.
III. eine Lösung von 20 Gew.-Teilen einer erfindungsgemäßen Verbindung I in einer Mischung aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl; durch feines Verteilen der Formulierung in Wasser erhält man eine Dispersion.
IV. eine wäßrige Dispersion aus 20 Gew.-Teilen einer erfindungsgemäßen Verbindung I, in einer Mischung aus 25 Gew.-Teilen Cyclohexanon, 65 Gew.-Teilen einer Mineralölfraktion vom Siedepunkt 210 bis 280 °C und 10 Gew.-Teilen des Anlagerungsproduktes von 40 mol Ethylenoxid an 1 mol Ricinusöl; durch feines Verteilen der Formulierung in Wasser erhält man eine Dispersion.
V. eine in einer Hammermühle vermahlene Mischung aus 20 Gew.-Teilen einer erfindungsgemäßen Verbindung I, 3 Gew.-Teilen des Natriumsalzes der Diisobutylnaphtalin-α-sulfonsäure, 17 Gew.-Teilen des Natriumsalzes einer Ligninsulfonsäure aus einer Sulfitablauge und 60 Gew.-Teilen pulverförmigem Kieselsäuregel; durch feines Verteilen der Mischung in Wasser erhält man eine Spritzbrühe;
VI. eine innige Mischung aus 3 Gew.-Teilen einer erfindungsgemäßen Verbindung I und 97 Gew.-Teilen feinteiligem Kaolin; dieses Stäubemittel enthält 3 Gew.-% Wirkstoff;
VII. eine innige Mischung aus 30 Gew.-Teilen einer erfindungsgemäßen Verbindung I, 92 Gew.-Teilen pulverförmigem Kieselsäuregel und 8 Gew.Teilen Paraffinöl, das auf die Oberfläche dieses Kieselsäuregels gesprüht wurde; diese Aufbereitung gibt dem Wirkstoff eine gute Haftfähigkeit;
VIII. eine stabile wäßrige Dispersion aus 40 Gew.-Teilen einer erfindungsgemäßen Verbindung I, 10 Gew.-Teilen des Natriumsalzes eines Phenosulfonsäure-harnstoff-formaldehyd-Kondensates, 2 Gew.-Teilen Kieselgel und 48 Gew.-Teilen Wasser, die weiter verdünnt werden kann;
IX. eine stabile ölige Dispersion aus 20 Gew.-Teilen einer erfindungsgemäßen Verbindung I, 2 Gew.-Teilen des Calciumsalzes der Dodecylbenzolsulfonsäure, 8 Gew.-Teilen Fettalkohol-polyglykol-ether, 2 Gew.-Teilen des Natriumsalzes eines Phenolsulfonsäure-harnstoff-formaldehyd-Kondensates und 68 Gew.-Teilen eines paraffinischen Mineralöls;
X. eine in einer Hammermühle vermahlene Mischung aus 10 Gew.-Teilen einer erfindungsgemäßen Verbindung I, 4 Gew.-Teilen des Natriumsalzes der Diisobutylnaphthalin-α-sulfonsäure, 20 Gew.-Teilen des Natriumsalzes einer Ligninsulfonsäure aus einer Sulfitablauge, 38 Gew.-Teilen Kieselsäuregel und 38 Gew.-Teilen Kaolin. Durch feines Verteilen der Mischung in 10 000 Gew.-Teilen Wasser erhält man eine Spritzbrühe, die 0,1 Gew.% des Wirkstoffs enthält.

Die Verbindungen I werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Die Aufwandmengen liegen je nach Art des gewünschten Effektes zwischen 0,02 und 3 kg Wirkstoff pro ha, vorzugsweise bei 0,1 bis 1 kg/ha.

Bei der Saatgutbehandlung werden im allgemeinen Wirkstoffmengen von 0,001 bis 50 g, vorzugsweise 0,01 bis 10 g je Kilogramm Saatgut benötigt.

Die Aufwandmenge an Wirkstoff für die Bekämpfung von Schädlingen beträgt unter Freilandbedingungen 0,02 bis 10, vorzugsweise 0,1 bis 2,0 kg/ha Wirkstoff.

Die Verbindungen I, allein oder in Kombination mit Herbiziden oder Fungiziden, können auch noch mit weiteren Pflanzenschutzmitteln gemischt gemeinsam ausgebracht werden, beispielsweise mit Wachstumsregulatoren oder mit Mitteln zur Bekämpfung von Schädlingen oder Bakterien. Von Interesse ist ferner die Mischbarkeit mit Düngemitteln oder mit Mineralsalzlösungen, welche zur Behebung von Ernährungs- und Spurenelementmängeln eingesetzt werden.

Die Pflanzenschutz- und Düngemittel können zu den erfindungsgemäßen Mitteln im Gewichtsverhältnis 1:10 bis 10:1 zugesetzt werden, gegebenenfalls auch erst unmittelbar vor der Anwendung (Tankmix). Beim Vermischen mit Fungiziden oder Insektiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'propylen-bis-dithiocarbamat), N,N'-Polypropylen-bis-(thiocarbamoyl)-disulfid; Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthals°ure-di-isopropylester;
heterocyclische Substanzen, wie 2-Heptadecyl-2-imidazolin-acetat, 2,4-Dichlor-6-(o-chloranilino)-s-triazin, O,O-Diethyl-phthalimidophosphonothioat, 5-Amino-1-β-[bis-(dimethylamino)-phosphinyl]-3-phenyl-1,2,4-triazol, 2,3-Dicyano-1,4-dithioanthrachinon, 2-Thio-1,3-dithiolo-β-[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthiophthalimid, N-Dichlorfluormethylthio-N',N'-dimethyl-N-phenylschwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iodbenzoesäure-anilid, N-Formyl-N-morpholin-2,2,2-trichlorethylacetal, Piperazin-1,4-diylbis-(1-(2,2,2-trichlor-ethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclodedecyl-morpholin bzw. dessen Salze, N-[3-(p-tert.-Butylphenyl)-2-methylpropyl]-cis-2,6-dimethylmorpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol 1-[2-(2,4-Dichlorphenyl)-4-n-propyl-1,3-dioxolan-2-ylethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-l-yl)-2-butanol, α-(2-Chlorphenyl)-α-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol,
sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methylester, N-(2,6-Dimethylphenyl)-N-chloracetyl-D,L-2-aminobutyrolacton, DL-N-(2,6-Dimethylphenyl)-N- (phenylacetyl) -alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl]-1,3-oxazolidin-2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in der anschließenden Tabelle mit physikalischen Daten aufgeführt.
1. α-Keto-2-(1-phenyltriazolyl-3) -acetiminoxymethyl) -phenylessigsäuremethylester-trans-O-methyloxim (nicht erfindungsgemäß) 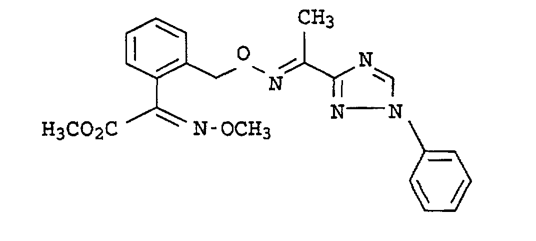
   a) 1-Phenyl-3-acetyltriazol
      Eine Mischung von 2 g (11,3 mmol) Brenztraubensäureamid-1-phenylhydrazon (DE 2750813) und 20 ml Triethylorthoformiat wurde 1 Stunde auf 100°C erhitzt. Anschließend wurde die Reaktionsmischung bei vermindertem Druck eingeengt und der verbleibende Rückstand wurde mit Methyl-t-butylether ausgerührt und abgesaugt. Man erhielt 1,9 g (90 %) der Titelverbindung als farblosen Festkörper.
      ¹H-NMR (DMSO-d₆; δ in ppm):
      9,5 (s,1H, Triazolyl), 7,95 (d,2H, Phenyl); 7,6 (m,2H, Phenyl); 7,5 (m,1H, Phenyl); 2,65 (s,3H,CH₃).
   b) 1-Phenyl-3-acetyltriazol-oxim
      Eine Mischung von 28 g (0,15 mol) 1-Phenyl-3-acetyltriazol (Beispiel 1a), 12 g (0,19 mol) Hydroxylamin-hydrochlorid und 16 g (0,2 mol) Natriumacetat zu 225 ml Ethanol/Wasser 2:1 wurde über Nacht bei Raumtemperatur (= 25°C) gerührt.
      Anschließend wurde die Reaktionsmischung mit Wasser verdünnt und mit Methylenchlorid extrahiert. Die vereinigten organischen Phasen wurden eingeengt. Der Rückstand kristallisierte und wurde mit Methyl-t-butylether/Hexan ausgerührt. Man erhielt 27,5 g (91 %) der Titelverbindung als farblosen Festkörper.
      ¹H-NMR (DMSO-d₆; δ in ppm):
      11,75 (s,1H,OH); 9,45 (s,1H,Triazolyl); 8,0 (d,2H, Phenyl); 7,7 (t,2H, Phenyl); 7,55 (t,1H, Phenyl; 2,35 (s,3H,CH₃).
   c) α-Keto-2-((1-Phenyltriazolyl-3)-acetiminoxymethyl)-phenylessigsäuremethylester-trans-O-methyloxim
      Eine Mischung von 2,8 g (9,8 mmol) α-Keto-2-brommethylphenylessigsäuremethylester-trans-O-methyloxim (EP 254426), 2 g (9,9 mmol) 1-Phenyl-3-acetyltriazol-oxim (Beispiel b) und 2 g (14 mmol) K₂CO₃ in 25 ml Dimethylformamid wurde 3 Tage bei Raumtemperatur (= 25°C) gerührt. Anschließend wurde die Reaktionsmischung mit Wasser verdünnt und die wäßrige Phase wird dreimal mit Methyl-t-butylether extrahiert. Die vereinigten organischen Phasen wurden über MgSO₄ getrocknet und eingeengt. Der Rückstand wurde säulenchromatographisch mit Cyclohexan/Essigester-Gemischen gereinigt. Das Produkt kristallisierte und wurde mit Methyl-t-butylether/Hexan ausgerührt. Man erhielt 2,2 g (55 %) der Titelverbindung als farblosen Festkörper (Fp. = 123°C).
      ¹H-NMR (DMSO-d₆; δ in ppm):
      8,5 (s,1H,Triazolyl); 7,7 (d,2H,Phenyl); 7,3-7,6 (m,6H,Phenyl); 7,2 (m,1H,Phenyl); 5,25 (s,2H,OCH₂); 4,05 (s,3H,OCH₃); 3,85 (s,3H,OCH₃); 2,35 (s,3H,CH₃).
2. α-Keto-2-((1-phenyltriazolyl-3)-acetiminoxymethyl)-phenylessigsäure-N-methylamid-trans-O-methyloxim (nicht erfindungsgemäß)

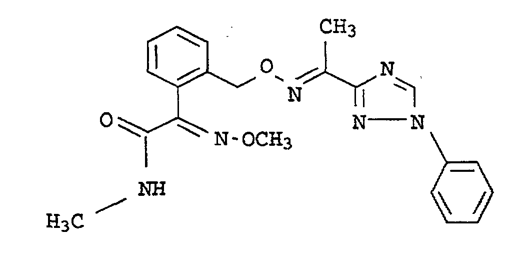

Eine Mischung von 1,5 g (3,7 mmol) a-Keto-2-((1-phenyltriazolyl-3)-acetiminoxymethyl)-phenylessigsäuremethylester-trans-O-methyloxim (Beispiel 1) und 20 ml wäßriger Methylamin-Lösung (40 %ig) wurde ca. 2 Stunden bei 50°C gerührt. Anschließend verdünnte man die Reaktionsmischung mit Wasser und extrahierte die wäßrige Phase mit Methylenchlorid. Die vereinigten organischen Phasen, wurden mit Wasser extrahiert, über MgSO₄ getrocknet und eingeengt. Man erhielt 1,2 g (77 %) der Titelverbindung als beigen Festkörper (Fp. = 60°C).
¹H-NMR (DMSO-d₆; δ in ppm):
8,55 (s,1H,Triazolyl); 7,7 (d,2H,Phenyl); 7,5 (m,3H,Phenyl); 7,4 (m,3H),Phenyl); 7,2 (m,1H,Phenyl); 6,9 (s,breit,1H,NH); 5,25 (s,2H,OCH₂); 3,95 (s,3H,OCH₃); 2,95 (d,3H,n-CH₃); 2,35 (s,3H,CH₃).

**Tabelle**

| **Nr.** | **R**^{**1**} | **R**^{**2**} _{**n**} | **R**^{**3**} | **R**^{**4**} | **R**^{**5**} | **phys. Daten**^{**a**} |
|---|---|---|---|---|---|---|
| 05 | C(CO₂CH₃)=CHCH₃ | H | CH₃ | H | C₆H₅ | 100 |
| 06 | C (CO₂CH₃) =CHCH₃ | H | CH₃ | CH₃ | C₆H₅ | 86 |
| 20 | C (CO₂CH₃) =CHCH₃ | H | CH₃ | CF₃ | 2-Cl-C₆H₄ | 79 |
| 21 | C(CO₂CH₃)=CHCH₃ | H | CH₃ | CF₃ | 3-Cl-C₆H₄ | 103 |
| 22 | C(CO₂CH₃)=CHCH₃ | H | CH₃ | CF₃ | 4-Cl-C₆H₄ | 1715, 1501, 1255, 1195, 1152, 1090, 1035, 1025, 1008, 759 |
| 23 | C(CO₂CH₃)=CHCH₃ | H | CH₃ | CF₃ | 2-CH₃-C₆H₄ | 3,7(s,3H); 2,4(s,3H); 2,1(s,3H); 1,65(d,3H) |
| 24 | C(CO₂CH₃)=CHCH₃ | H | CH₃ | CF₃ | 3-CH₃-C₆H₄ | 1716, 1498, 1254, 1212, 1187, 1151, 1103, 1036, 1009, 759 |
| 25 | C(CO₂CH₃)=CHCH₃ | H | CH₃ | CF₃ | 4-CH₃-C₆H₄ | 1716, 1514, 1304, 1254, 1195, 1151, 1101, 1030, 1009, 759 |

| | | | | | | |
|---|---|---|---|---|---|---|
| a: Fp (°C) : ¹H-NMR (ppm); IR (cm⁻¹) | | | | | | |

### Beispiele zur Wirkung gegen Schadpilze

Die fungizide Wirkung der Verbindungen der Formel I ließ sich durch folgende Versuche zeigen:

Die Wirkstoffe wurden als 20 %-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole) aufbereitet und entsprechend der gewünschten Konzentration mit Wasser verdünnt.

### Wirkung gegen Pyricularia oryzae (Reisbrand)

Reiskeimlinge (Sorte: "Tai Nong 67") wurden mit der Wirkstoffaufbereitung tropfnaß gespritzt (Aufwandmenge: 63 ppm). Nach 24 Stunden wurden die Pflanzen mit einer wäßrigen Sporensuspension des Pilzes *Pyricularia oryzae* besprüht und 6 Tage bei 22-24°C bei einer relativen Luftfeuchtigkeit von 95-99% bewahrt. Die Beurteilung erfolgte visuell.

In diesem Test zeigten die mit den erfindungsgemäßen Verbindungen Nrs. 5, 6, 22 und 25 behandelten Pflanzen einen Befall von 15% und weniger.

**Wirkung gegen Erysiphe graminis var.** tritici *(Weizenmehltau)*

Blätter von Weizenkeimlingen (Sorte "Frühgold") wurden zunächst mit der wäßrigen Aufbereitung der Wirkstoffe behandelt (Aufwandmenge: 63 ppm). Nach ca. 24h wurden die Pflanzen mit Sporen des Weizenmehltaus (*Erysiphe graminis var*. *tritici*) bestäubt. Die so behandelten Pflanzen wurden anschließend für 7 Tage bei 20-22°C und einer relativen Luftfeuchtigkeit von 75-80% inkubiert. Anschließend wurde das Ausmaß der Pilzentwicklung ermittlelt.

In diesem Test zeigten die mit den erfindungsgemäßen Verbindungen Nrs. 20, 22, 23, 24 und 25 behandelten Pflanzen einen Befall von 15% und weniger während die unbehandelten (Kontroll-) Pflanzen zu 70% befallen waren.

### Beispiele zur Wirkung gegen tierische Schädlinge

Die Wirkung der Verbindungen der allgemeinen Formel I gegen tierische Schädlinge ließ sich durch folgende Versuche zeigen:
Die Wirkstoffe wurden
   a) als 0,1 %-ige Lösung in Aceton oder
   b) als 10 %-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole)
   aufbereitet und entsprechend der gewünschten Konzentration mit Aceton im Fall von a) bzw. mit Wasser im Fall von b) verdünnt.

Nach Abschluß der Versuche wurde die jeweils niedrigste Konzentration ermittelt, bei der die Verbindungen im Vergleich zu unbehandelten Kontrollversuchen noch eine 80 - 100 %-ige Hemmung bzw. Mortalität hervorriefen (Wirkschwelle bzw. Minimalkonzentration).

## Patentansprüche

1. Iminooxybenzylverbindungen der Formel I 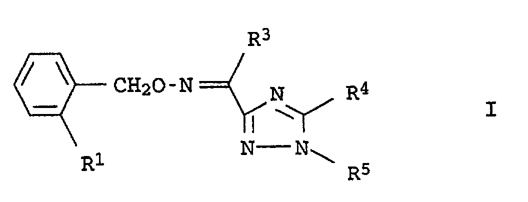 in der die Substituenten die folgende Bedeutung haben:
R¹ C(CO₂R^{a})=CHR^{b};
R^{a},R^{b} C₁-C₄-Alkyl;
R³ Cyano, Halogen, C₁-C₄-Alkyl, Cyclopropyl, Trifluormethyl, Methoxy, Ethoxy oder Methylthio;
R⁴ Wasserstoff, Cyano, Halogen, C₁-C₄-Alkyl, C₁-C₄-Halogenalkyl, C₁-C₄-Alkoxy, C₁-C₄-Halogenalkoxy oder C₁-C₄-Alkylthio;
R⁵ Phenyl, welches ein bis drei der folgenden Reste tragen kann: Cyano, Halogen, C₁-C₄-Alkyl oder CRⁱⁱⁱ=NOR^{iv}; oder welches durch C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₁-C₆-Alkoxy, C₂-C₆-Alkenyloxy, C₂-C₆-Alkinyloxy, C₁-C₆-Halogenalkyloxy, C₂-C₆-Halogenalkenyloxy, C₂-C₆-Halogenalkinyloxy substituiert sein kann;
Rⁱⁱⁱ Wasserstoff, C₁-C₆-Alkyl, C₂-C₆-Alkenyl oder C₂-C₆-Alkinyl;
R^{iv} C₁-C₆-Alkyl, C₂-C₆-Alkenyl, C₂-C₆-Alkinyl oder Phenyl-C₁-C₆-alkyl.

2. Verfahren zur Herstellung der Verbindungen der Formel I gemäß Anspruch 1, **dadurch gekennzeichnet, daß** man eine Benzylverbindung der Formel II 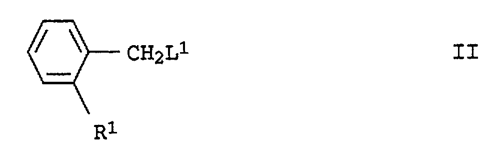 in der L¹ für eine Abgangsgruppe steht, mit einem Oxim der Formel III 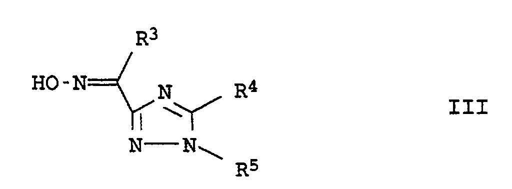 oder dessen Salz umsetzt.

3. Verfahren zur Herstellung der Verbindungen der Formel I gemäß Anspruch 1, **dadurch gekennzeichnet, daß** man einen Hydroxylaminether der Formel IV 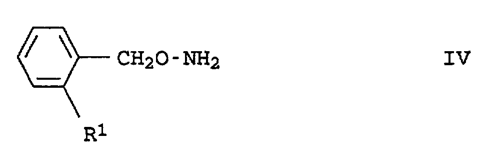 in einem inerten organischen Lösungsmittel mit einem Triazolylketon der Formel V 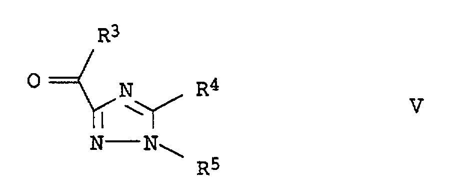 umsetzt.

4. Verbindungen der Formel VI 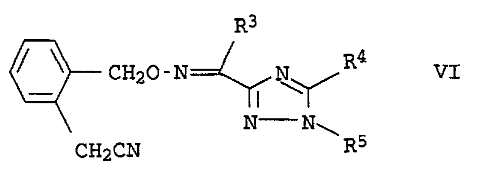 in der die Substituenten die in Anspruch 1 gegebene Bedeutung haben.

5. Verbindungen der Formel VII 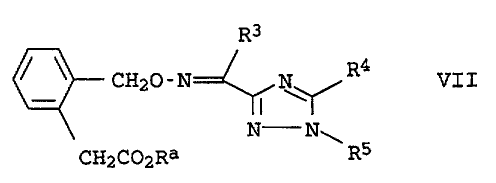 in der die Substituenten die in Anspruch 1 gegebene Bedeutung haben.

6. Verbindungen der Formel VIII 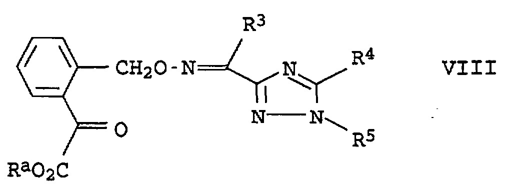 in der die Substituenten die in Anspruch 1 gegebene Bedeutung haben.

7. Zur Bekämpfung von Schädlingen oder Schadpilzen geeignetes Mittel, enthaltend einen festen oder flüssigen Trägerstoff und eine Verbindung der allgemeinen Formel I gemäß Anspruch 1.

8. Verwendung der Verbindungen I gemäß Anspruch 1 zur Herstellung eines zur Bekämpfung von Schädlingen oder Schadpilzen geeigneten Mittels.

9. Verfahren zur Bekämpfung von Schadpilzen, **dadurch gekennzeichnet, daß** man die Pilze oder die vor Pilzbefall zu schützenden Materialien, Pflanzen, Boden oder Saatgüter mit einer wirksamen Menge einer Verbindung der allgemeinen Formel I gemäß Anspruch 1 behandelt.

10. Verfahren zur Bekämpfung von Schädlingen, **dadurch gekennzeichnet, daß** man die Schädlinge oder die von ihnen zu schützenden Materialien, Pflanzen, Boden oder Saatgüter mit einer wirksamen Menge einer Verbindung der allgemeinen Formel I gemäß Anspruch 1 behandelt.

## Claims

1. An iminooxybenzyl compound of the formula I 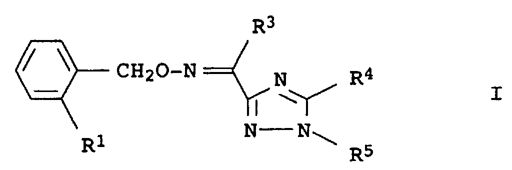 where the substituents have the following meanings:
R¹ is C(CO₂R^{a})=CHR^{b};
R^{a},R^{b} are C₁-C₄-alkyl;
R³ is cyano, halogen, C₁-C₄-alkyl, cyclopropyl, trifluoromethyl, methoxy, ethoxy or methylthio;
R⁴ is hydrogen, cyano, halogen, C₁-C₄-alkyl, C₁-C₄-haloalkyl, C₁-C₄-alkoxy, C₁-C₄-haloalkoxy or C₁-C₄-alkylthio;
R⁵ is phenyl which can carry one to three of the following radicals: cyano, halogen, C₁-C₄-alkyl or CRⁱⁱⁱ = NOR^{iv}; or which can be substituted by C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₁-C₆-alkoxy, C₂-C₆-alkenyloxy, C₂-C₆-alkynyloxy, C₁-C₆-haloalkyloxy, C₂-C₆-haloalkenyloxy, C₂-C₆-haloalkynyloxy;
Rⁱⁱⁱ is hydrogen, C₁-C₆-alkyl, C₂-C₆-alkenyl or C₂-C₆-alkynyl;
R^{iv} is C₁-C₆-alkyl, C₂-C₆-alkenyl, C₂-C₆-alkynyl or phenyl-C₁-C₆-alkyl.

2. A process for the preparation of the compounds of the formula I as claimed in claim 1, which comprises reacting a benzyl compound of the formula II 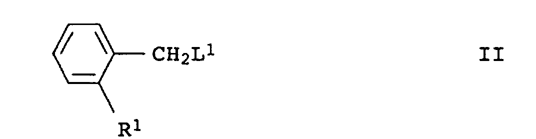 where L¹ is a leaving group, with an oxime of the formula III 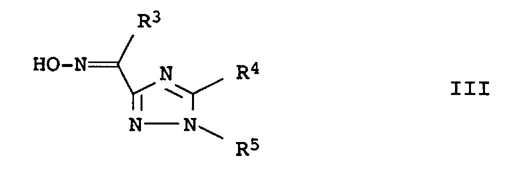 or a salt thereof.

3. A process for the preparation of the compounds of the formula I as claimed in claim 1, which comprises reacting a hydroxylamine ether of the formula IV 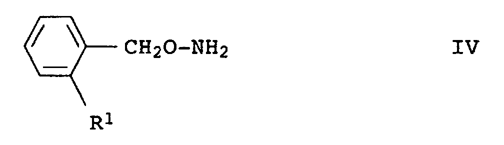 with a triazolyl ketone of the formula V 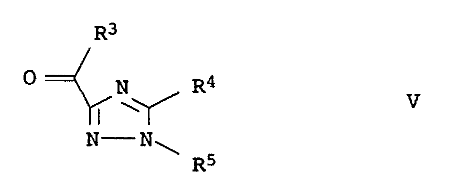 in an inert organic solvent.

4. A compound of the formula VI 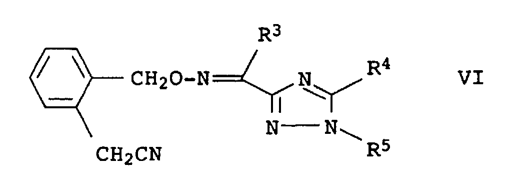 where the substituents have the meanings given in claim 1.

5. A compound of the formula VII 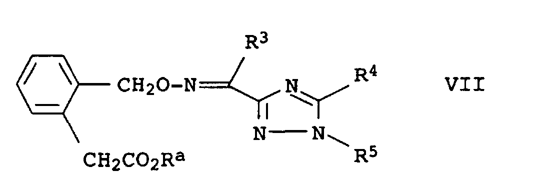 where the substituents have the meanings given in claim 1.

6. A compound of the formula VIII 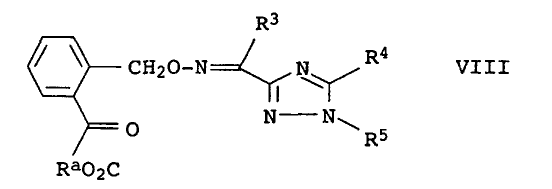 where the substituents have the meanings given in claim 1.

7. A composition which is suitable for controlling animal or fungal pests, comprising a solid or liquid carrier and a compound of the general formula I as claimed in claim 1.

8. The use of the compounds I as claimed in claim 1 for the preparation of a composition which is suitable for controlling animal pests or fungal pests.

9. A method of controlling fungal pests, which comprises treating the fungi or the materials, plants, soil or seed to be protected against fungal infection with an effective amount of a compound of the general formula I as claimed in claim 1.

10. A method of controlling animal pests, which comprises treating the animal pests or the materials, plants, soil or seed to be protected against them with an effective amount of a compound of the general formula I as claimed in claim 1.

## Revendications

1. Composés d'iminooxybenzyle de formule I 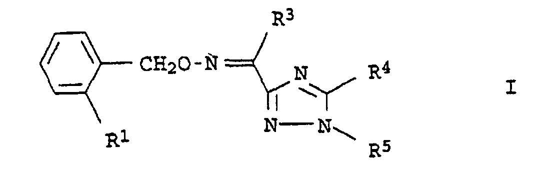 dans laquelle les substituants ont la signification suivante:
R¹ C(CO₂R^{a})=CHR^{b};
R^{a},R^{b} alkyle en C₁-C₄;
R³ cyano, halogéno, alkyle en C₁-C₄, cyclopropyle, trifluorométhyle, méthoxy, éthoxy ou méthylthio;
R⁴ hydrogène, cyano, halogéno, alkyle en C₁-C₄, halogénoalkyle en C₁-C₄, alcoxy en C₁-C₄, halogénoalcoxy en C₁-C₄ ou alkylthio en C₁-C₄;
R⁵ phényle, qui peut porter un à trois des restes suivants: cyano, halogéno, alkyle en C₁-C₄ ou CRⁱⁱⁱ=NOR^{iv}; ou qui peut être substitué par des groupes alkyle en C₁-C₆, alcényle en C₂-C₆, alcynyle en C₂-C₆, halogénoalkyle en C₁-C₆, halogénoalcényle en C₂-C₆, halogénoalcynyle en C₂-C₆, alcoxy en C₁-C₆, alcényloxy en C₂-C₆, alcynyloxy en C₂-C₆, halogénoalkyloxy en C₁-C₆, halogénoalcényloxy en C₂-C₆, halogénoalcynyloxy en C₂-C₆;
Rⁱⁱⁱ hydrogène, alkyle en C₁-C₆, alcényle en C₂-C₆ ou alcynyle en C₂-C₆;
R^{iv} alkyle en C₁-C₆, alcényle en C2-C6, alcynyle en C2-C6 ou phényl-alkyle(C₁-C₆).

2. Procédé pour la préparation des composés de formule I selon la revendication 1, **caractérisé par le fait qu'**on fait réagir un composé benzylique de formule II 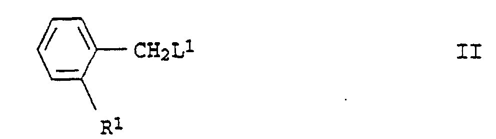 dans laquelle L¹ désigne un groupe éliminable, avec une oxime de formule III 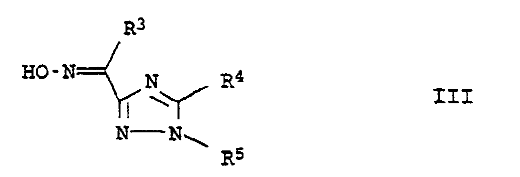 ou un sel de celle-ci.

3. Procédé pour la préparation des composés de formule I selon la revendication 1, **caractérisé par le fait qu'**on fait réagir un hydroxylamino-éther de formule IV 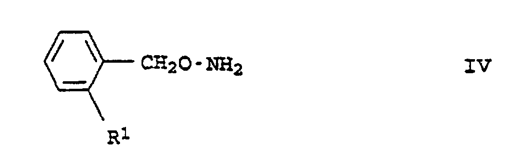 dans un solvant organique inerte avec une triazolylcétone de formule V

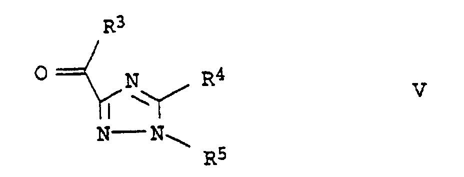

4. Composés de formule VI 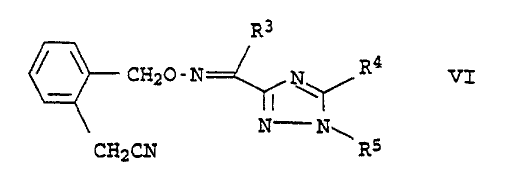 où les substituants ont la signification indiquée dans la revendication 1.

5. Composés de formule VII 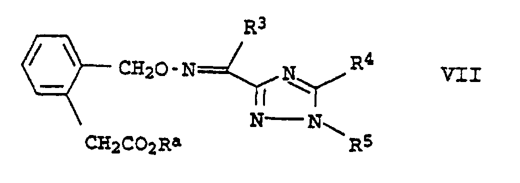 où les substituants ont la signification indiquée dans la revendication 1.

6. Composés de formule VIII 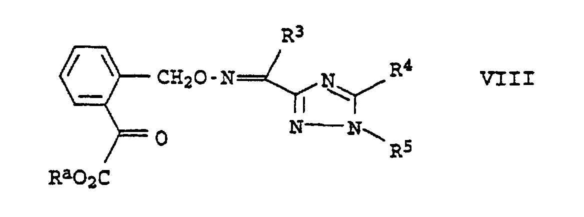 où les substituants ont la signification indiquée dans la revendication 1.

7. Produit approprié pour la lutte contre les parasites ou les champignons nuisibles, contenant un support solide ou fluide et un composé de formule générale I selon la revendication 1.

8. Utilisation des composés I selon la revendication 1 pour la préparation d'un produit approprié pour la lutte contre les parasites ou les champignons nuisibles.

9. Procédé pour la lutte contre les champignons nuisibles, **caractérisé par le fait qu'**on traite les champignons ou les matériaux, plantes, sols ou semences à protéger contre l'attaque par les champignons avec une quantité efficace d'un composé de formule générale I selon la revendication 1.

10. Procédé pour la lutte contre les parasites, **caractérisé par le fait qu'**on traite les parasites ou les matériaux, plantes, sols ou semences à protéger contre eux avec une quantité efficace d'un composé de formule générale I selon la revendication 1.
